# EUROPEAN PATENT APPLICATION

(11) **EP 2 700 361 A1**
(43) Date of publication of application: **26.02.2014**
(21) Application number: 12774110.6
(22) Date of filing: 21.04.2012
(51) Int. Cl.: A61B 6/00, A61B 19/00

(54) **METHOD FOR QUICKLY AND PRECISELY CALIBRATING MEDICAL IMAGING COMPONENT AFTER CHANGING POSITION**

(30) Priority: 22.04.2011 CN 201110102204
(71) Applicant: Weidu Medical Systems, Inc., Chongqing 401121 (CN)
(72) Inventor: MU, Zhiping, Chongqing 400060 (CN)
(74) Representative: Stuttard, Garry Philip
(86) International application number: PCT/CN2012/074487
(87) International publication number: WO 2012/142972

(57) **Abstract**

A method for quickly and precisely calibrating a medical imaging component after position change, comprising: placing a imaging component at a reference position, calibrating, precisely by a first measuring system, the reference position and obtaining a precise calibration value of the reference position; at the same time, performing, by a second measuring system, a second calibration for the reference position of the imaging component and obtaining a reference calibration value of the reference position; measuring, by the second measuring system, the current position of the imaging component and obtaining a reference calibration value of the current position; obtaining a precise calibration value of the current position via calculation based on the precise calibration value of the reference position, the reference calibration value of the reference position, and the reference calibration value of the current position.

## Description

The present invention relates to the field of medical imaging technology, and in particular, to a method for quickly and precisely calibrating a medical imaging component after position change.

### Description of the Related Art

At present, medical imaging systems are widely used in medical diagnosis and treatment, such as image guidance systems for diagnosis, operation, interventional treatment, and radiotherapy.

The precise geometric position of the imaging system in the reference coordinate system often needs to be obtained when the medical imaging system is collecting images of patients. For example, in an Image Guided Radiation Therapy (IGRT) system, the system determines the current position of the patient (with respect to a reference position) by comparing the patient images obtained in real time with the patient reference images obtained beforehand. For this purpose, the positions of imaging components, including X-Ray tubes and detectors, must be precisely calibrated, which is complicated and time consuming.

The above requirements impose strict restrictions on the geometric position of the imaging system. For example, in a medical imaging system and image guided system for radiotherapy, multiple projections from different angles are required. Usually, the imaging system (or imaging component) could only move along a fixed track or path, as it does in a CT system. When the initial position of the imaging system changes, the coordinate position after the change needs to be known precisely to ensure the application value of the obtained projected images. For the majority of imaging or treatment systems, the coordinates after position change are obtained through the mechanical movements of the imaging systems along their paths. In the prior arts, the above method requires high precision of mechanical parts in the imaging equipment, and during the process, the mechanical wear may greatly affect the precision of the measurement results. To sum up, the prior arts are complicated, highly demanding, and time consuming. Furthermore, calibration of reference position often employs a certain type of ionizing radiation, which is harmful to human health.

Therefore, constrains on geometric position subsequently affect clinical outcomes of the systems, like sacrificing the optimal imaging distance and angle, thus failing to realize the optimal imaging quality, time consumption and dosage.

### SUMMARY OF THE INVENTION

To address the above defects in the prior arts, the present invention is directed to prolviding a method for quickly and precisely calibrating medical imaging components after changing position, which lowers mechanical requirements on equipment and avoids the effect of mechanical wear on measurement results.

The technical embodiments in the present invention to solve the above technical problems are implemented in the following way:

A method for quickly and precisely calibrating medical imaging components after position change employs medical imaging equipment containing a first measuring system and a second measuring system for measurement, and the precise calibrating process comprises the following steps:

1) Take the initial position of the medical imaging component as a reference position, obtain precise calibration values by the first measuring system through precise calibrating of the reference position and obtain reference calibration values by the second measuring system through a second calibrating of the reference position.

2) After a medical imaging component of the medical imaging equipment moves, the new position of the medical imaging component is regarded as the current position. Then the second measuring system is employed to obtain reference calibration values of the current position through measurement.

3) Precise calibration values of the current position are obtained through calculation based on the precise calibration values of the reference position and the reference calibration values of the reference position obtained in step 1), together with the reference calibration values of the current position obtained in step 2), thus achieving precise calibration after the imaging component moves.

The current position of the above imaging component may be obtained by measuring the position deviation of the current position from the reference position. The methods for measuring the position deviation of the imaging component comprise, but are not limited to, the following four:

1. Place one or more mechanical sensors on the imaging component, and measure mechanical parameters during the movement of the imaging component recorded by the mechanical sensor and calculate the position deviation of the component.

2. Attach multiple tracers to the imaging component; the second measuring system is an auxiliary optical imaging system (visible light or infrared); measure tracers' position change in the image acquired by the auxiliary optical imaging system to determine the position deviation.

3. Attach energy storage elements to the imaging component; these energy storage elements can be activated by electromagnetic (EM) pulses and then emit EM signals independently. The second measuring system measures EM signals or signal variance (during the movement of imaging component) emitted from the energy storage elements, and calculates the position deviation of the imaging component.

4. Attach speakers or transducers to the imaging component, wherein these speakers or transducers can emit acoustic wave, ultrasound wave, or continuous pulse signals in a predetermined manner, measure these signals by the second measuring system and then calculate the position deviation of the imaging component.

In the present invention, once the first measuring system completes precise calibration of the reference position, the first measuring system is regarded as a current calibration system (high precision, but of low speed and high cost), and the subsequent position change of the imaging component can be calibrated by the second measuring system. The second measuring system measures only moving components of the imaging system, unlike the measuring system (the first measuring system) in the prior art that has to measure the whole imaging system; the first measuring system has to measure all components that affect imaging geometry; meanwhile, the calibration coordinates or origin of the second measuring system may differ from the first measuring system. Since the second measuring system is fast and easy to use, the whole measuring and calibrating process is fast and easy to implement, and thus the precise post-movement position can be obtained via calculation, thereby achieving fast and precise post-movement calibration. This method allows the current medical imaging systems to move in a greater space, and eliminates the limitations where the imaging component could only be either fixed or move along a fixed track, thus improving the clinical outcomes of the imaging system.

Since the first measuring system usually employs a certain ionizing radiation for precise calibration, whereas the second measuring system does not require ionizing radiation, the implementation of this method expands clinical applications and benefits human health by abandoning the ionizing radiation.

This method is especially suitable to the medical imaging systems in (clinical) applications where precise position information of imaging components is required, and one or more components of imaging system are movable (non-fixed). Measuring the position deviation of the imaging component between current position and reference position by the second measuring system needs to be done in a relatively short period of time.

### BRIEF DESCRIPTION OF THE DRAWING

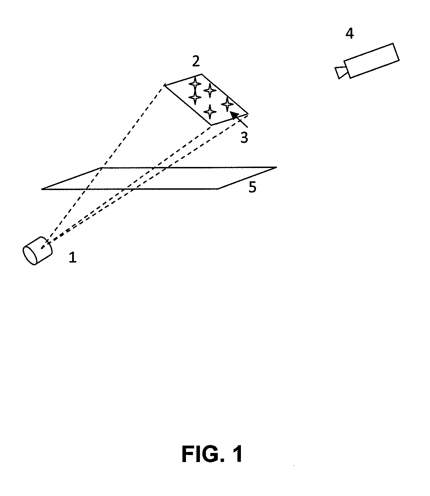

FIG. 1 is a schematic view of measurement according to an embodiment of the present invention.

### DETAILED DESCRIPTION OF THE EMBODIMENTS

The present invention is further illustrated in the following with reference to the accompanying drawings and embodiments. It is obvious that the embodiments to be described are only a part rather than all of the embodiments of the present invention. All other embodiments obtained by persons skilled in the art based on the embodiments of the present invention without paying any creative efforts shall fall within the protection scope of the present invention.

A method for quickly and precisely calibrating medical imaging components after position change employs medical imaging equipment comprising a first measuring system and a second measuring system for measurement, wherein the precise calibration process comprises the following steps:

1. Take the initial position of the medical imaging component as a reference position, obtain precise calibration values by the first measuring system through precise calibrating of the reference position and obtain reference calibration values by the second measuring system through a second calibrating of the reference position.

2. After a medical imaging component of the medical imaging equipment moves, the new position of the medical imaging component is regarded as the current position. Then the second measuring system is employed to obtain reference calibration values of the current position through measurement.

3. Precise calibration values of the current position are obtained through calculation based on the precise calibration values of the reference position and the reference calibration values of the reference position obtained in step 1, together with the reference calibration values of the current position obtained in step 2, thus achieving precise calibration after the imaging component moves.

Specifically, the method for quickly and precisely calibrating medical imaging components after position change is as follows. 1. Place the imaging component at a convenient position for application. This position is called a reference position. Then the first measuring system precisely calibrates the reference position and obtains precise calibration values of the reference position; at the same time, the second measuring system performs a second calibration of the reference position of the imaging component and obtains reference calibration values of the reference position; 2. After the medical imaging component moves in response to a specific application, the new post-movement position is called a current position and the second measuring system is employed to obtain reference calibration values of the current position. 3. Precise calibration values of the current position are obtained via calculation based on the precise calibration values and the reference calibration values of the reference position, together with the reference calibration values of the current position, thus achieving precise calibration of the post-movement imaging component. For the specific calculations, refer to the prior arts, which will be not be elaborated herein.

In the present invention, the imaging components are to form images in medical imaging system, such as X-Ray tubes and imagers in an X-Ray system.

The current position of an imaging component (shift and/or rotation) is obtained by measuring the deviation of the current position from the reference position. The methods to measure the position deviation of the imaging component comprise, but are not limited to, the following four:

1. Place one or more mechanical sensors, such as an acceleration sensor, angular acceleration sensor, or gyroscope, on the imaging component, and by the second measuring system, measure and record mechanical parameters during the movement, and calculates the position deviation before and after the movement.

2. Attach multiple tracers to the imaging component, and measure tracers' position change in the image acquired by the auxiliary optical (visible light or infrared) imaging system to determine the position deviation. The auxiliary optical imaging system is the second measuring system.

3. Implement by measuring EM signals. Attach one or more small energy storage elements to the imaging component. These energy storage elements can be activated by EM pulses and then emit EM signal independently after the EM pulses end. They work in a way similar to transponders in airplanes. The second measuring system measures and analyzes the EM signals emitted by the energy storage elements after being activated, and calculates the position deviation (from the reference position) of the imaging component.

4. Implement by measuring acoustic or ultrasound signals. Attach one or more micro speakers or transducers to the imaging component. These speakers or transducers can emit acoustic wave, ultrasound wave, or continuous pulses signals in a predetermined manner. The second measuring system measures and analyzes these signals to calculate the position deviation (from the reference position) of the imaging component.

A specific embodiment of measurement is given below to illustrate this method, where optical imaging systems are adopted to measure the position of components of an X-Ray imaging system.

As shown in FIG. 1, the medical imaging system is an X-Ray system, comprising an X-Ray tube 1 and an imager 2. The position of imager in this embodiment is flexible. Multiple optical tracers 3 are attached to the back or sides of the imager. The optical camera 4 is the auxiliary system (the second measuring system) that measures the position change of the imager 2. The application process of the present invention is as follows.

First, place the X-Ray tube 1 and the imager 2 at an appropriate position with respect to treatment bed 5, wherein this position is the reference position. This reference position is measured and precisely calibrated to obtain its imaging geometry parameters. Then fix the optical camera 4 to a position where it has a good view of the tracers 3 on the imager 2, and precisely calibrate it to determine its imaging geometry parameters. The precise calibration of the optical camera enables it to measure, the position deviation as well as the tracers' position values. The second measuring system may directly measure the post-movement position of the imaging component, albeit the origin of the measurement coordinates may be different from that of the first measuring system.

While the imager 2 is at the reference position, use the optical camera 4 to observe (from the captured image) and then take a current position of tracers 3 as the starting position.

When the imager 2 moves from its reference position, use camera 4 to observe and measure (from the captured image) the post-movement position of tracers 3. Compared with the foregoing starting position of the tracers, the geometry parameters of post-movement position of imager 2 can be calculated. (The precision of geometry parameters in this position is identical with the first step.)

In this solution, conventional methods could be used for geometry parameter measurement and precise calibration of the X-Ray imaging system and the optical imaging system, such as measuring with ruler and angle meter or calibrating with computer vision methods. All these methods, however, require relatively complicated and highly precise procedures, which are time consuming, taking hours to complete. The optical camera could be either visible or infrared light, and the tracers could be either active or passive infrared tracers, or makers or lines that stand out sharply against the background. Many existing imaging processing and computer vision methods may be adopted to arrange tracers, extract their positions from optical images and subsequently calculate the geometry parameters of the imager.

Regarding the method to measure the position the tracers with infrared camera, one could refer to the manual of Polaris system (NDI, Ontario, Canada). This system locates infrared tracer balls with two infrared cameras, achieving the accuracy of 0.25 mm within a relatively wide range. The positioning accuracy could be further improved if higher resolution cameras are employed. If 3 or more tracer balls are affixed to the back of the imager, the imager's position deviation could be calculated by measuring the tracer balls' starting positions and post-movement positions (or position change). The new position parameters of the imager can be obtained based on the position deviation combined with the reference position of the imager.

The scheme of adopting acoustic positioning systems is similar to the foregoing scheme. Affix (one or more) acoustic speakers to the imager (functionality similar to the tracer), and place one or more microphones to appropriate locations (functionality similar to the optical camera). The acoustic speakers emit acoustic pulses with different frequencies and amplitudes following a certain pattern; by measuring and analyzing the frequency, amplitude and phase of acoustic pulses received by these microphones, speaker positions can be calculated. Similar to the foregoing optical system, the imager's position deviation could be calculated by measuring the starting position and the post-movement position (or position change) of the speakers. Combined with the reference position of the imager, the new position parameters of the imager can be obtained.

The scheme of adopting EM wave positioning systems is also similar to the foregoing schemes. Affix (one or more) transponders to the imager (functionality similar to the tracers), and place an active antenna array to an appropriate location (functionality similar to the optical camera). The active antenna array transmits certain excitation EM pulses following a specific pattern; after the excitation pulses end, the transponders emit EM wave signals via its energy storage mechanism; these signals are received by the active antenna array, and the position of every transponder with respect to the active antenna array can be calculated. Similar to the foregoing optical system scheme, the imager's position deviation can be calculated by measuring the starting position and the post-movement position (or position change) of the transponders. Combined with the reference position of the imager, the new position parameters of the imager can be derived. The principle of this scheme can also be found in the manuals and documents of Calypso Medical's (Now a Varian company) 4D localization system. J.M. Balter, et. al., "Accuracy of a wireless localization system for radiotherapy" Int J Radiat Oncol Biol Phys., 2005 Mar 1; 61(3): 933-7. The difference lies in that the Calypso system requires implantation of transponders (called beacons) into patients for tumor localization, whereas in this invention, the transponders are attached to imaging components.

The method adopting mechanical sensors is slightly different. For example, acceleration sensors and angular acceleration sensors can be attached to the imager. Set the readings to zero when the imager is located at the reference position, then these sensors record a series of acceleration and angular acceleration values while the imager is in motion. Perform integration to obtain speed and angular speed, and then perform an additional integration to derive the moving distance and angles; combined with the reference position of the imager, the new position parameters of the imager can be derived.

In the above schemes, the characteristic of this invention is to utilize an auxiliary calibration or measuring method (such as the optical camera in this embodiment) to measure the post-movement geometry parameters of the imaging components (X-Ray imager in this embodiment). Compared with the precise calibration method of the primary imaging system (the X-Ray system in this embodiment), this auxiliary method is faster and easy to use while abandoning radiation. If this method is employed, the position of the X-Ray imaging components do not have to be either fixed or highly restricted, more flexibility in system configurations is allowed, thereby improving system performance and user friendliness.

The foregoing schemes are merely one embodiment of this invention, which allows varied alternations, such as including one or more cameras in the optical system to broaden the view.

## Claims

1. A method for quickly and precisely calibrating a medical imaging component after position change, wherein the medical imaging equipment employed comprises a first measuring system and a second measuring system, and the precise calibration process comprises:
1) taking an initial position of the medical imaging component as a reference position, obtaining, by the first measuring system, precise calibration values through precise calibrating of the reference position, and meanwhile obtaining, by the second measuring system, reference calibration values through a second calibrating of the reference position;
2) taking a new position of the medical imaging component after movement as a current position and measuring, by the second measuring system, to obtain reference calibration values of the current position through measurement;
3) calculating pprecise calibration values of the current position based on the precise calibration values of the reference position and the reference calibration values of the reference position obtained in step 1), together with the reference calibration values of the current position obtained in step 2), to achieve precise calibration after the imaging component moves.

2. The method according to claim 1, wherein the calibrating quickly and precisely medical imaging components after position change, comprises:
determining the current position of the imaging component by measuring position deviation of the current position from the reference position.

3. The method according to claim 2, wherein the measuring method of position deviation of the imaging component comprises placing one or more mechanical sensors on the imaging component, and measuring, by the second measuring system, mechanical parameters during the movement of the imaging component recorded by the mechanical sensor and calculating the position deviation of the component.

4. The method according to claim 2, wherein the measuring method of position deviation of the imaging component comprises attaching multiple tracers to the imaging component, the second measuring system being an auxiliary optical imaging system, and measuring tracers' position change in the image acquired by the auxiliary optical imaging system to determine the position deviation.

5. The method according to claim 2, wherein the measuring method position deviation of the imaging component comprises attaching one or more small energy storage elements to the imaging component; these energy storage elements may be activated by electromagnetic (EM) pulses and then emit EM signals independently after the EM pulses end; during the process of movement, the second measuring system measures EM signals emitted by the energy storage elements after being activated and EM signal variance, and calculates the position deviation of the imaging component.

6. The method according to claim 2, wherein the measuring method of position deviation of the imaging component comprises attaching speakers or transducers to the imaging component, measuring, by the second measuring system, these signals and then calculating the position deviation of the imaging component; these speakers or transducers are configured to emit acoustic wave, ultrasound wave, or continuous pulse signals in a predetermined manner.
